(19) Europäisches Patentamt
European Patent Office
Office européen des brevets

(11) Publication number: **0 355 409 B1**

(12) # EUROPEAN PATENT SPECIFICATION

(45) Date of publication of patent specification: **09.11.94**

(51) Int. Cl.[5]: **A61K 37/64**, A61K 31/535, A61K 31/445, A61K 31/425, A61K 31/40

(21) Application number: **89113351.4**

(22) Date of filing: **20.07.89**

(54) **Use of prolylendopeptidase inhibitors in the treatment of AIDS.**

(30) Priority: **25.07.88 JP 185259/88**

(43) Date of publication of application:
**28.02.90 Bulletin 90/09**

(45) Publication of the grant of the patent:
**09.11.94 Bulletin 94/45**

(84) Designated Contracting States:
**AT BE CH DE ES FR GB GR IT LI LU NL SE**

(56) References cited:
**EP-A- 0 154 353**

**CHEMICAL ABSTRACTS, vol. 78, no. 24, 18 June 1973, Columbus, OH (US); p. 460, no. 160117g***

**CELL, vol. 54, no. 3, 29 July 1988, Cell Press; J.SCHNEIDER et al., pp. 363-368***

**CZECHOSLOV. MED., vol. 11, no. 4, 1988; Z.LOJDA, pp. 181-194***

(73) Proprietor: **KABUSHIKI KAISHA YAKULT HONSHA**
**1-19, Higashishinbashi 1-chome**
**Minato-ku Tokyo 105 (JP)**

(72) Inventor: **Takatsuki, Kiyoshi**
**1-16-3-54, Toroku**
**Kumamoto-shi Kumamoto (JP)**
Inventor: **Hattori, Toshio**
**1-14-10-104, Shin-oe**
**Kumamoto-shi Kumamoto (JP)**
Inventor: **Tsuru, Daisuke**
**10-16-502, Shiratori-cho**
**Nagasaki-shi Nagasaki (JP)**
Inventor: **Yoshimoto, Tadashi**
**2-29-10, Nameishi**
**Nagasaki-shi Nagasaki (JP)**

(74) Representative: **Wächtershäuser, Günter, Prof. Dr.**
**Patentanwalt**
**Tal 29**
**D-80331 München (DE)**

**EP 0 355 409 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

i) Field of the Invention:

This invention relates to preventive and curative agents for acquired immune deficiency syndrome (AIDS) and anti-HIV agents, and more specifically to high-safety preventive and curative agents for AIDS and high-safety anti-HIV agents.

ii) Description of the Background Art:

AIDS is a viral infectious disease whose first victim was reported in 1981 in the United States. As of May, 1988, 85,000 cases were reported around the world, centering at the United States and including 97 patients in Japan [AIDS Information File (24), Nihon Iji Shimpo 3379, 119 (1989)]. In each AIDS patient, the number of T-helper cells which play a key role in the immune system of the human being has been reduced significantly so that an immunological deficient state has occurred. As a result, the patient develops a malignant tumor such as Kaposi's sarcoma, Pneumocystis carinii or candidiasis or other opportunistic infections. Furthermore, neurological symptoms such as dementia due to spreading of the infection to the nervous tissue is also observed frequently.

At first, there was a view that AIDS was a special disease limited to male homosexuals and drug abusers (addicts). However, its transmission routes have been being uncovered recently. For example, mother-to-infant infections, infections from the use of contaminated blood or blood derivatives, and infections of medical workers through accidents have been reported. It has also been known that infections take place by heterosexual intercourse.

AIDS has become a serious social problem in many years led by the United States, because in spite of its very high death rate, namely, no report of cure, no therapeutic method has been established and measures for the prevention of infection are insufficient.

In the meantime, a great deal of work has been being conducted with respect to AIDS, led by the discovery and identification of the HIV (human immunodeficiency virus) as an infectious virus in 1983. Substantial knowledge has hence been accumulated in a short period of time. Nevertheless, no satisfactory results have been achieved. For example, it is very difficult to develop a vaccine because the AIDS virus is prone to mutation. On the other hand, azidothymidine (AZT) analogous to nucleic acids has already been recognized effective as a curative drug. It has however been found that this drug has strong toxicity such as inhibition of bone marrow function and is unable to promise complete cure.

It has hence been desired to provide a medicine effective for the prevention of infection of T cells from the AIDS virus thereby developing a measure for the prevention of AIDS infection and also for the treatment of AIDS.

### SUMMARY OF THE INVENTION

Under these circumstances, the present inventors have proceeded with an investigation on the mechanism of infection of T cells from HIV. As a result, it has been found that certain inhibitors against prolylendopeptidase, which is a sort of peptidase and is hereinafter abbreviated as "PEP", have excellent anti-HIV activities and are effective for the prevention of infection form AIDS, leading to completion of this invention. It has been known that benzyloxycarbonylamino ester piperides of the type used by the present invention have analgesic and antiacetylcholine activities (JP-A-7301078).

### DETAILED DESCRIPTION OF THE INVENTION AND PREFERRED EMBODIMENTS

This invention therefore provides the use of a PEP inhibitor as defined in Claim 1 in the manufacture of preventive and curative agents for AIDS and anti-HIV agents.

PEP inhibitors useful in the present invention are those represented by the following formula (I):

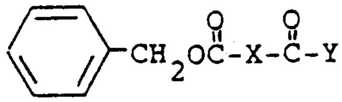

(I)

wherein X means

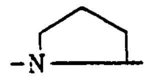 or 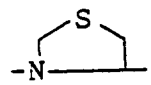 ,

and Y denotes

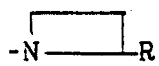 , 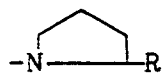 , 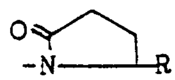 , 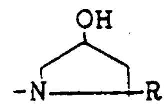 , 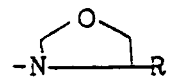 ,

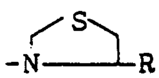 , 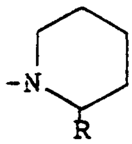 , 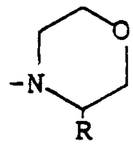 , 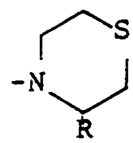 ,

in which R is hydrogen, formyl, carboxyl or $C_{1-4}$ alcoxycarbonyl. As the lower alcoxycarbonyl represented by R groups such as methoxycarbonyl, ethoxycarbonyl and propoxycarbonyl are preferred.

Some of these compounds have already been known from Japanese Patent Application Laid-Open No. 188317/1985 and page 396, Abstracts of Lectures at the 108th Annual Conference of the Pharmaceutical Society of Japan (1988).

The compounds of the formula (I) can be prepared by a general peptide synthesis process, for example, in accordance with the following reaction formula:

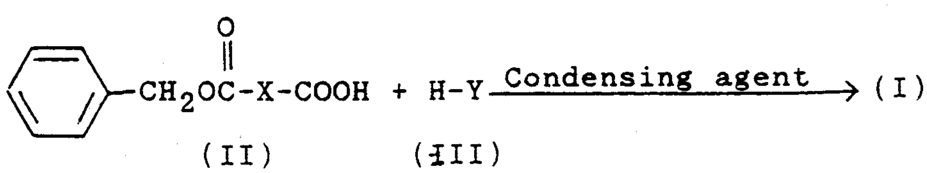


(II) (III)

wherein x and Y have the same meanings as defined above.

Namely, the compounds (I) can be obtained by reacting amino acid derivatives (II) and amino acids or their related compounds (III), both of which contain benzyloxycarbonyl-blocked amino groups as moieties corresponding to X and Y respectively, in the presence of a condensing agent.

No particular limitation is imposed on the condensing agent so long as it is employed for the condensation of amino acids. Dicyclohexylcarbodiimide may be mentioned by way of example. It is preferable to conduct the reaction at a temperature of from -10 °C to 50 °C in an inert solvent. Examples of the solvent include ether solvents such as tetrahydrofuran and dioxane, aromatic hydrocarbons such as benzene, and alkyl halides such as dichloromethane and chloroform.

The PEP inhibitors being used as AIDS-curing agents or anti-HIV agents, can be formed into suitable preparation forms, for example, tablets, capsules, soft capsules, powders, injections, and plasters. For these preparation forms, excipients, dissolution aids and the like can be used. Administration can be effected by a variety of methods, for example, various injections such as intravenous injection, subcutaneous injection, intramuscular injection and intramedullary injection, oral administration, and diadermic administration. Particularly preferred are oral administration and intravenous administration. The preferable dialy dosage is 5-900 mg as PEP inhibitor in oral administration and 1-500 mg as PEP inhibitor in intravenous administration. The dosage can however be changed depending on the conditions of AIDS, the administration method, etc., so that they can be administered outside the above ranges.

The AIDS preventive and curative agents according to this invention inhibit the syncytium forming phenomenon characteristic to intercellular infection form HIV.

When HIV-infected T cells and non-infected T cells are mixed in a test tube, the infected T cells and non-infected T cells undergo fusion and form giant cells. This "syncytium forming phenomenon" has already been known to be observed upon their mixing. This phenomenon is characteristic to intercellular infection from HIV and is regarded as an evidence for HIV infection of T cells.

When these PEP inhibitors are added to a mixed culture system of HIV-infected T cells and non-infected T cells, the above-mentioned syncytium forming phenomenon is inhibited effectively. Further, a certain correlation is observed between PEP inhibitory activities and syncytium formation inhibitory activities.

As has been described above, the preventive and curative agents of this invention prevent and cure AIDS by inhibiting HIV infection from infected cells to non-infected cells, in other words, by anti-HIV activities.

The syncytium forming phenomenon is considered to be a cell fusion based on the interactions of external envelope glycoprotein (gp 120) of HIV infected cells and cell membrane receptors for HIV in uninfected cells. The preventive and curative agents of this invention, which inhibit the formation of syncytia, are high-specificity peptidase inhibitors. They are hence believed to inhibit viral encapsulation and even the digestion of cell membranes upon infiltration of HIV into T cells. This effect is believed to prevent direct infection of T cells from HIV.

In addition, the PEP inhibitors of this invention represented by formula (I) have high safety as already been reported in Japanese Patent Application Laid-Open No. 188317/1985, and EP-A-0154353.

Therefore, the preventive and curative agents of this invention have excellent anti-HIV activities and high safety and are useful for the prevention and cure of AIDS.

EXAMPLES:

The AIDS preventive and curative agents of this invention will hereinafter be described in further detail by the following examples.

Preparation Example 1:

Preparation of N-benzyloxycarbonyl-prolyl-prolinal

(1) Condensation Reaction

N-benzyloxycarbonyl-proline (22.1 g) was dissolved in tetrahydrofuran (200 ml), followed by the addition of N-hydroxysuccinimide (11.5 g) and dicyclohexylcarbodiimide (20.6 g) under cooling with sodium chloride and ice. The resultant mixture was stirred at 4 °C for 21 hours. The reaction mixture was filtered, and the solvent was distilled off from the filtrate to obtain a semi-solid matter. The semi-solid matter was recrystallized from isopropyl alcohol to obtain a white solid matter. Tetrahydrofuran (450 ml) was added to the solid matter to dissolve the latter, followed by the addition of L-prolinol (8.2 g). The thus-obtained mixture was stirred for 72 hours at room temperature. After distilling off the tetrahydrofuran, the residue was dissolved in ethyl acetate (300 ml). The resultant solution was washed with 1 M $Na_2CO_3$ (100 ml), dried over anhydrous $Na_2SO_4$ followed by evaporation to dryness to obtain N-benzyloxycarbonyl (hereinafter referred to as Z)-prolyl-prolinol.

(2) Oxidation Reaction

The resultant white solid matter was added with 1-(3-dimethylaminopropyl)-3-ethylcarbodiimide hydrochloride (40.5 g) and redistilled dimethyl sulfoxide (150 ml), followed by stirring for 10 minutes. Thereafter, 6.3 ml of a 2 M solution of anhydrous $H_3PO_4$ in dimethyl sulfoxide was added, followed by stirring for additional 2 hours. After terminating the reaction with a 1 M potassium phosphate buffer (350 ml, pH: 7.5), the reaction mixture was extracted with ethyl acetate (200 ml) and then dried over anhydrous $Na_2SO_4$. The ethyl acetate was distilled off and the residue was dissolved in ethanol (200 ml). $NaHSO_3$ (200 g) dissolved in water (350 ml) was then added, followed by vigorous mixing for 10 minutes. After concentration of the ethanol to about 350 ml, the unreacted compounds were removed twice with 100-ml portions of ethyl ether. Solid $Na_2CO_3$ was then added until the pH was raised to 9. Five minutes later, the resultant mixture was extracted twice with 100-ml portions of ethyl ether. The ethyl ether was distilled off, and the residue was recrystallized from the ethyl ether to obtain the title compound. Yield: 3.4 g (10.3%).

Preparation Examples 2-22:

The following compounds were prepared in a similar manner to Preparation Example 1 using Z-proline or Z-thioproline and various cyclic amines as starting materials. The materials were subjected to a

condensation reaction and, when required, a subsequent oxidation reaction.

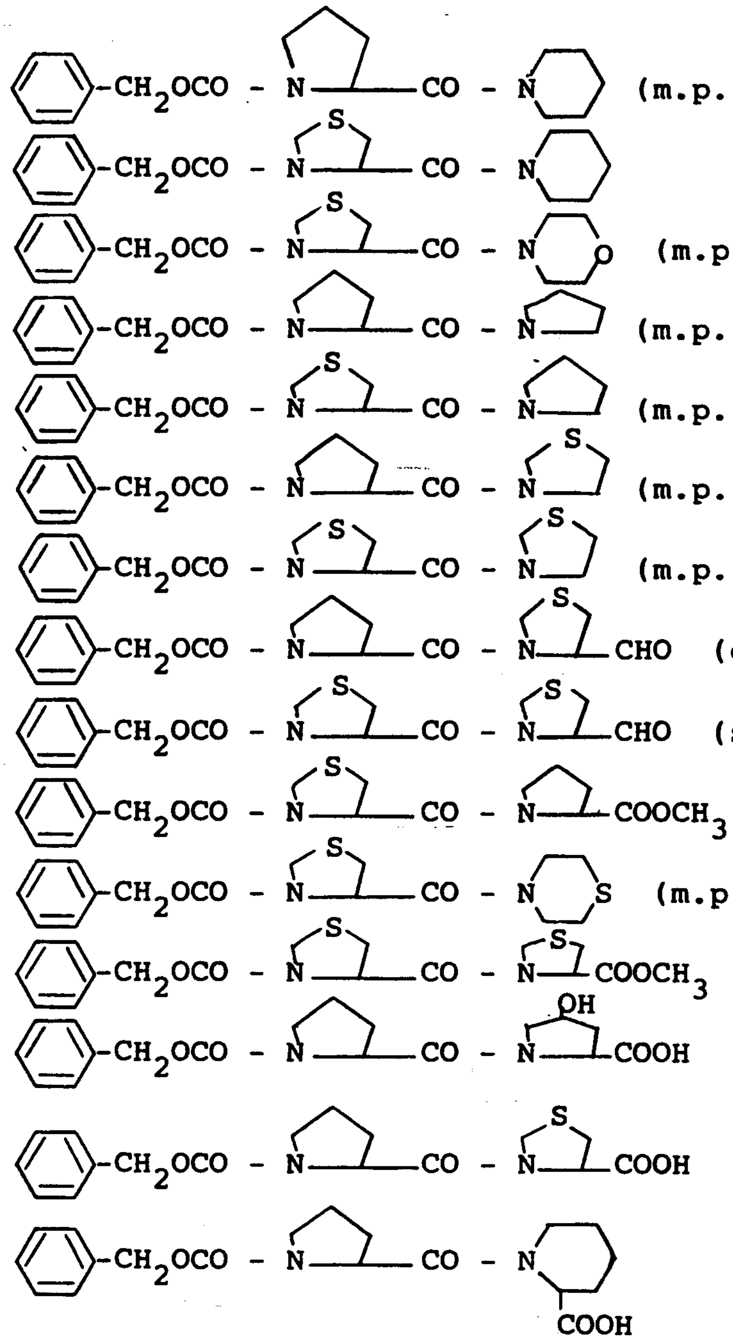

2. (m.p.: 90-92°C)

3.

4. (m.p.: 114-115°C)

5. (m.p.: 129-131°C)

6. (m.p.: 88-90°C)

7. (m.p.: 112-114°C)

8. (m.p.: 73-75°C)

9. (oily material)

10. (semi-solid)

11. (oily material)

12. (m.p.: 120-123°C)

13. (m.p.: 102-104°C)

14. (m.p.: 217-218°C, decomposed)

15. (m.p.: 183-185°C, decomposed)

16. (m.p.: 66-69°C)

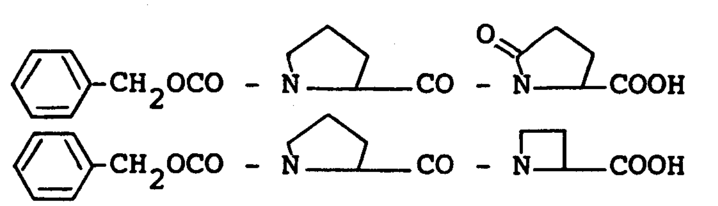

17. (m.p.: 133-135°C)

18. (m.p.: 146-148°C)

19. 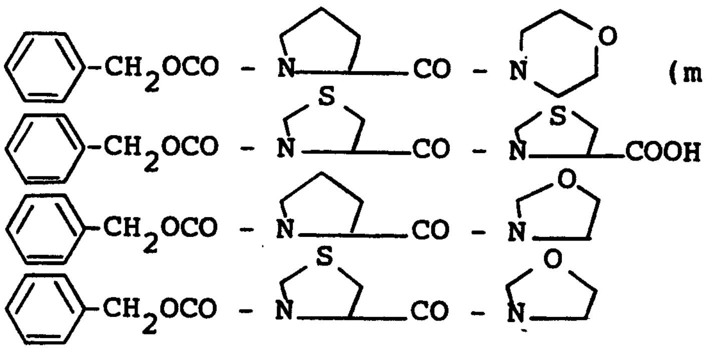 (m.p.: 148-150°C)

20. (m.p.: 188-189°C)

21.

22.

Example 1:

LAV-1 infected CEM cells (CEM/LAV-1, 2 x $10^4$) and MOLT4 Clone 8 (1 x $10^5$) were used as HIV-infected cells and non-infected cells, respectively. Those two types of cells were cultured in 200 µl/well of RPMI1640 medium added with 10% FCS in a 96-well microplate. The PEP inhibitors of this invention and comparative product, which are shown in Table 1, were separately added. The cells were cultured at 37°C for 18 hours in a capneic incubator. Formation of syncytia was observed. Results are shown in Table 1. The PEP inhibitions ($IC_{50}$; nM) of the tested compounds are also shown in Table 1. The PEP inhibitory action was measured according to the following method.

Measurement of PEP Inhibitory Action

Z-Gly-Pro-β-naphtyl amide was used as a substrate and the inhibitory action against PEP derived from bovine brain was measured.

(Method)

0.7 ml of 20 mM-tris HCl buffer (pH 7.0) containing 10 mM of EDTA and 10 mM of 2-mercaptoethanol was added with 100 µl of PEP (about 0.14 u/ml) and 100 µl of a test solution which had been prepared to have a predetermined concentration (0, $10^{-9}$ - $10^{-4}$M), and subjected to preincubation at 37°C for 5 minutes. To this was added various concentrations of a substrate which had been dissolved in 100 µl of 40% dioxane followed by incubation at 37°C for 15 minutes to proceed an exzymatic reaction. The reaction was terminated by the use of 25% trichloroacetic acid. The solution was centrifugally separated at 3,000 rpm for 10 minutes to obtain 0.5 ml of a supernatent, to which was added 0.5 ml of 0.1% nitrous acid, and 3 minutes after, 0.05% N-(1-naphtyl)ethylendediaminedihydrochloride ethanol solution. After allowing the mixture to stand at 37°C for 25 minutes, the absorbance at 570 nm was measured. The following equation was used to obtain the enzyme activity at each concentration and, based on the activity, 50% inhibition concentration ($IC_{50}$) was obtained.

Enzyme activity unit (µmol/min/ml)
= ΔOD x 0.42 x dilution ratio

Table 1

| P E P Inhibitors | Inhibition of Syncytia Formation | | | | | PEP Inhibition $IC_{50}$ (nM) |
|---|---|---|---|---|---|---|
| | 3 mM | 1 mM | 300 μM | 100 μM | 30 μM | |
| 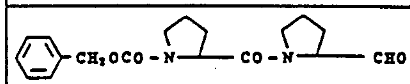 | +++ | +++ | ++ | + | ± | 0.74 |
| 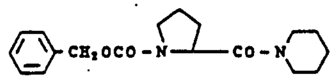 | | ± | | | | $>10^6$ |
| 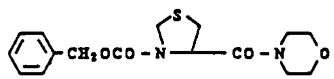 | + | ± | ± | ± | ± | 540 |
| 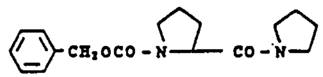 | ++ | + | ± | ± | ± | 540 |
| 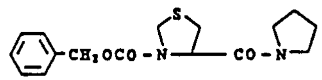 | ++ | + | ± | ± | | 160 |
| 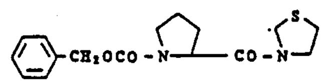 | | ++ | | | | 4.6 |
| 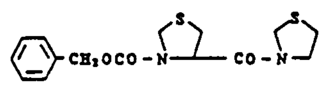 | | +++ | | | | 0.14 |
| 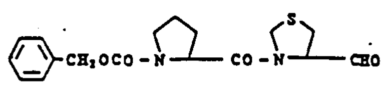 | | ++ | | | | 1.0 |
| 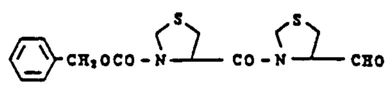 | | +++ | | | | 0.04 |
| 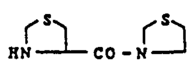 · HCl (comparative product) | | − | | | | $>10^6$ |

Rating standard:

-: Absolutely no inhibition of syncytium formation.

±: Slight inhibition of syncytium formation.

+: Partial inhibition of syncytium formation.

++: Substantial inhibition of syncytium formation.

+++: Complete inhibition of syncytium formation.

From the above results, it is understood that the PEP inhibitors of this invention can inhibit the formation of syncytia and their inhibitory effects increase with their concentrations.

**Claims**

**Claims for the following Contracting States : AT, BE, CH, DE, FR, GB, IT, LI, LU, NL, SE**

**1.** Use of a prolylendopeptidase inhibitor which is a compound represented by the following formula (I):

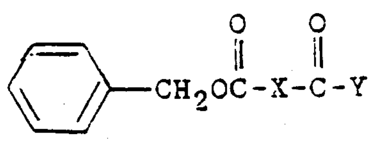 (I)

wherein X means

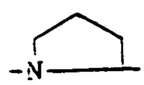 or 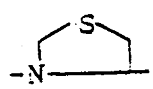 ,

and Y denotes

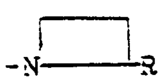 , 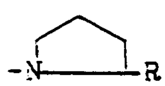 , 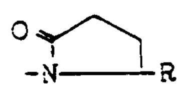 , 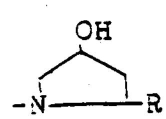 , 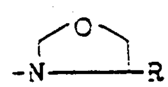 ,

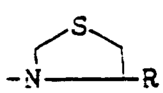 , 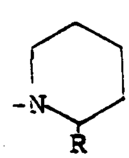 , 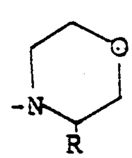 , 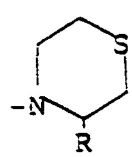 ,

in which R is hydrogen, formyl, carboxyl or $C_{1-4}$ alcoxycarbonyl in the manufacture of a preventive and curative agent for acquired immune deficiency syndrome.

**2.** Use of the compound recited in Claim 1 in the manufacture of an anti-HIV agent.

**Claims for the following Contracting States : ES, GR**

**1.** Process to prepare a peptide derivative with prolylendopeptidase inhibitory activity, useful as preventive and curative agent for acquired immune deficiency syndrome, of formula (I):

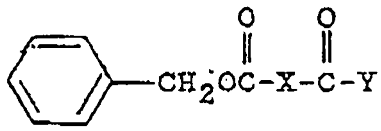

(I)

wherein X means

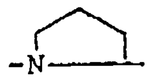 or 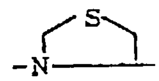 ,

and Y denotes

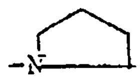 ,

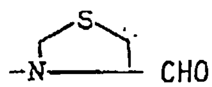 or 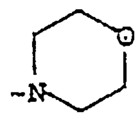

which comprises reacting an amino acid derivative of formula (II):

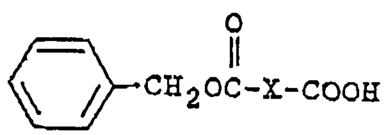

(II)

with another amino acid derivative of formula (III):

H - Y (III)

wherein X and Y have the same meanings as defined above, and carrying out the reaction in the presence of a condensing agent at a temperature between -10 ° C and 50 ° C and in an inert solvent.

**2.** Process accoring to claim 1, wherein said condensing agent is dicyclohexylcarbodiimide.

**3.** Process according to claim 1, wherein said solvent is selected from ethers, aromatic hydrocarbons and alkyl halides.

**4.** Process according to claim 1, wherein said solvent is selected from tetrahydrofuran, dioxane, benzene,dichloromethane and chloroform.

**Patentansprüche**

**Patentansprüche für folgende Vertragsstaaten : AT, BE, CH, DE, FR, GB, IT, LI, LU, NL, SE**

**1.** Verwendung eines Prolylendopeptidase Inhibitors, der eine durch die folgende Formel (I) dargestellte Verbindung ist:

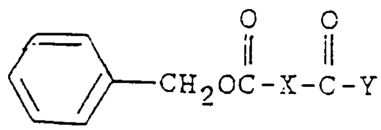 (I)

in der X

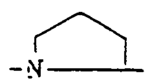 oder 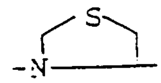

bedeutet und Y

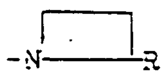 , 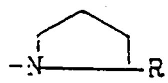 , 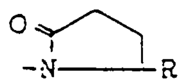 , 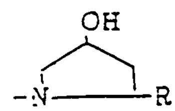 , 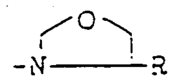 ,

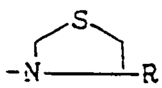 , 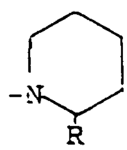 , 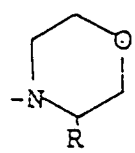 , 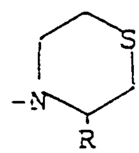

bedeutet, worin R Wasserstoff, Formyl, Carboxyl oder $C_{1-4}$-Alkoxycarbonyl ist, bei der Herstellung eines präventiven und kurativen Mittels für das erworbene Immundefizienz-Syndrom.

2. Verwendung der in Anspruch 1 aufgeführten Verbindung bei der Herstellung eines Anti-HIV-Mittels.

**Patentansprüche für folgende Vertragsstaaten : ES, GR**

1. Verfahren zur Herstellung eines Peptidderivats mit Prolylendopeptidase-hemmender Wirkung, nützlich als präventives und kuratives Mittel für das erworbene Immundefizienz-Syndrom, der Formel (I):

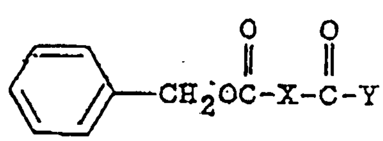 (I)

in der X

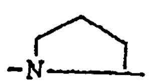 oder 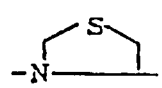

bedeutet und Y

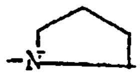 , 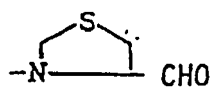 oder 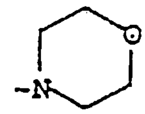

bedeutet, umfassend das Umsetzen eines Aminosäurederivats der Formel (II):

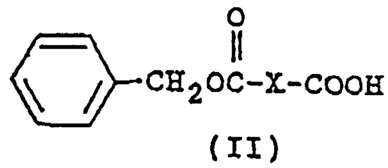

(II)

mit einem anderen Aminosäurederivat der Formel (III):

H - Y (III)

in der X und Y die gleichen wie oben definierten Bedeutungen aufweisen, und Durchführen der Umsetzung in Gegenwart eines Kondensationsmittels bei einer Temperatur zwischen -10 °C und 50 °C und in einem inerten Lösungsmittel.

**2.** Verfahren nach Anspruch 1, in dem das Kondensationsmittel Dicyclohexylcarbodiimid ist.

**3.** Verfahren nach Anspruch 1, in dem das Lösungsmittel aus Ethern, aromatischen Kohlenwasserstoffen und Alkylhalogeniden ausgewählt ist.

**4.** Verfahren nach Anspruch 1, in dem das Lösungsmittel aus Tetrahydrofuran, Dioxan, Benzol, Dichlormethan und Chloroform ausgewählt ist.

**Revendications**
**Revendications pour les Etats contractants suivants : AT, BE, CH, DE, FR, GB, IT, LI, LU, NL, SE**

**1.** Utilisation d'un inhibiteur de la prolylendopeptidase, qui est un composé représenté par la formule (I) suivante :

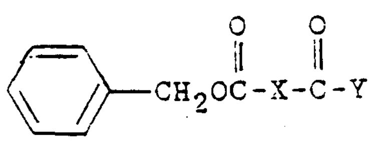

(I)

dans laquelle :
- X signifie

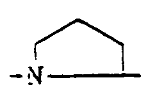 ou 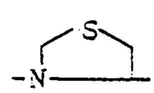 ;

et

- Y indique

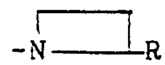 , 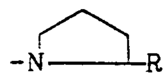 , 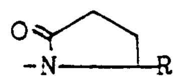 , 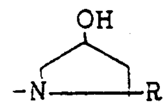 ,

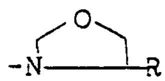 , 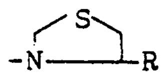 , 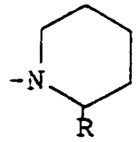 , 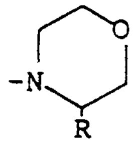 , 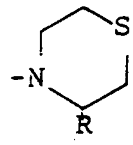 ,

où R représente hydrogène, formyle, carboxyle ou (alcoxy en $C_{1-4}$)carbonyle, dans la fabrication d'un agent pour la prévention et le traitement du syndrome immuno-déficitaire acquis.

**2.** Utilisation du composé tel que défini à la revendication 1, dans la fabrication d'un agent anti-HIV.

**Revendications pour les Etats contractants suivants : ES, GR**

**1.** Procédé de préparation d'un dérivé peptidique ayant une activité d'inhibition de la prolylendopeptidase, utile en tant qu'agent de prévention et de traitement du syndrome immuno-déficitaire acquis, ledit dérivé peptidique étant représenté par la formule (I) :

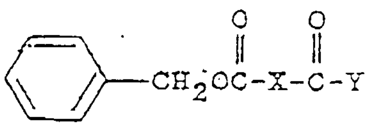 (I)

dans laquelle :

- X signifie

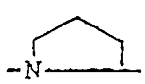 ou 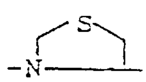 ;

et

- Y indique

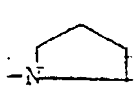 , 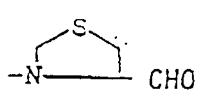 ou 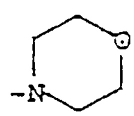

qui comprend les opérations consistant à

- faire réagir un dérivé d'acide aminé de formule (II) :

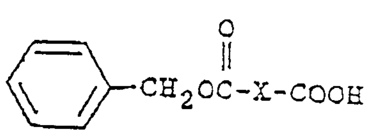

(II)

avec un autre dérivé d'acide aminé de formule (III) :

H - Y (III)

dans lesquelles X et Y ont les mêmes significations que celles définies ci-dessus ; et

- effectuer la réaction en présence d'un agent de condensation à une température comprise entre -10 ° C et 50 ° C et dans un solvant inerte.

2. Procédé selon la revendication 1, dans lequel ledit agent de condensation est le dicyclohexylcarbodiimide.

3. Procédé selon la revendication 1, dans lequel ledit solvant est choisi parmi les éthers, les hydrocarbures aromatiques et les halogénures d'alkyle.

4. Procédé selon la revendication 1, dans lequel ledit solvant est choisi parmi le tétrahydrofuranne, le dioxanne, le benzène, le dichlorométhane et le chloroforme.